(19) **Europäisches Patentamt**

**European Patent Office**

**Office européen des brevets**

(11) **EP 1 218 736 B1**

(12) **EUROPÄISCHE PATENTSCHRIFT**

(45) Veröffentlichungstag und Bekanntmachung des Hinweises auf die Patenterteilung:
**20.12.2006 Patentblatt 2006/51**

(21) Anmeldenummer: **00962475.0**

(22) Anmeldetag: **12.09.2000**

(51) Int Cl.:
**G01N 33/487** *(2006.01)* **G01N 27/403** *(2006.01)*

(86) Internationale Anmeldenummer:
**PCT/EP2000/008895**

(87) Internationale Veröffentlichungsnummer:
**WO 2001/027614 (19.04.2001 Gazette 2001/16)**

(54) **VORRICHTUNG ZUM MESSEN AN IN EINER FLÜSSIGEN UMGEBUNG BEFINDLICHEN ZELLEN**

DEVICE FOR TAKING MEASUREMENTS OF CELLS WHICH ARE CONTAINED IN A LIQUID ENVIRONMENT

DISPOSITIF POUR EFFECTUER DES MESURES SUR DES CELLULES SE TROUVANT DANS UN ENVIRONNEMENT LIQUIDE

(84) Benannte Vertragsstaaten:
**AT BE CH CY DE DK ES FI FR GB GR IE IT LI LU MC NL PT SE**

(30) Priorität: **08.10.1999 DE 19948473**

(43) Veröffentlichungstag der Anmeldung:
**03.07.2002 Patentblatt 2002/27**

(73) Patentinhaber:
• **CYTOCENTRICS AG**
**18059 Rostock (DE)**
• **Bayer Technology Services GmbH**
**51368 Leverkusen (DE)**

(72) Erfinder:
• **NISCH, Wilfried**
**72070 Tübingen (DE)**
• **STELZLE, Martin**
**72776 Reutlingen (DE)**
• **STETT, Alfred**
**72768 Reutlingen (DE)**
• **KRAHN, Thomas**
**58135 Hagen (DE)**
• **MÜLLER, Thomas**
**53225 Bonn-Beuel (DE)**

• **METHFESSEL, Christoph**
**42327 Wuppertal (DE)**

(74) Vertreter: **Duhme, Torsten**
**Witte, Weller & Partner,**
**Patentanwälte,**
**Postfach 10 54 62**
**70047 Stuttgart (DE)**

(56) Entgegenhaltungen:
EP-A- 0 627 621     EP-A- 0 689 051
WO-A-97/05922      DE-A- 19 646 505
DE-A- 19 712 309    DE-A- 19 827 957
DE-C- 19 841 337    US-A- 5 262 128

• **E. NEHER AND B. SAKMANN: "Single channel currents recorded from membrane of denervated frog muscle fibers" NATURE, Bd. 260, 1976, Seiten 799-802, XP000943702 London**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft eine Vorrichtung zum elektrischen Messen an in flüssiger Umgebung befindlichen Zellen, gemäß dem Oberbegriff von Anspruch 1.

Eine Vorrichtung der vorstehend genannten Art ist prinzipiell aus der DE 198 41 337 C1 bekannt.

Die aus der DE 198 41 337 C1 bekannte Vorrichtung dient zur intrazellulären Manipulation einer biologischen Zelle, die Erfassung von Messwerten ist in dieser Druckschrift nicht beschrieben.

Es ist allgemein bekannt, zum Untersuchen von biologischen Zellen sogenannte Mikroelektrodenanordnungen einzusetzen. Die Mikroelektrodenanordnungen dienen dabei z.B. zum Stimulieren der Zellen oder zum Ableiten von Potentialen. Die Untersuchungen können dabei in einer biologischen Umgebung durchgeführt werden oder auch in einer artifiziellen Umgebung. Die Anordnungen umfassen hierzu in einem Trägerkörper eine Vielzahl von Mikroelektroden, deren Abmessungen etwa in der Größenordnung der Zellen liegen, also im Bereich von einigen $\mu m$ bis einigen zehn $\mu m$. Eine Mikroelektrodenanordnung dieser allgemeinen Art ist z.B. aus der WO 97/05922 bekannt.

Bei herkömmlichen Mikroelektrodenanordnungen ist man mehr oder weniger auf den Zufall angewiesen, ob die eine oder die andere Zelle sich auf einer bestimmten Elektrode niederläßt oder nicht. In der Praxis lassen sich die Zellen im allgemeinen nur unter teilweiser Überdeckung auf einer Elektrode nieder, so daß die Stimulation der Zelle bzw. die Ableitung eines Zellpotentials auf diese Teilfläche beschränkt ist. Darüber hinaus sitzen die Zellen nur lose auf den Elektroden auf. Dies kann zu Problemen hinsichtlich des Abdichtwiderstandes zur Referenzelektrode führen. Auch können Zellen außerhalb des Bereichs einer Elektrode zu liegen kommen, so daß sie bei der Messung nicht erfaßt werden.

Bei einer aus der DE 197 12 309 A1 bekannten Mikroelektrodenanordnung werden diese Nachteile dadurch vermieden, daß die Zellen in Mikroküvetten eingefangen werden, an deren Boden sich eine Elektrode befindet. Die Elektrode ist mit einem zentralen Kanal versehen, in dem über geeignete Verbindungskanäle, die unterhalb der Elektroden verlaufen, ein Unterdruck erzeugt werden kann. Auf diese Weise ist es möglich, einzelne Zellen zielgerichtet an die Elektroden heranzuziehen und sie unter einem gewissen Kontaktdruck auf den Elektroden zu fixieren. Es können dann Messungen an den Elektroden, jedoch nur von deren Außenseite her, durchgeführt werden.

Aus einem anderen Fachgebiet, der sogenannten Patch-Clamp-Technik, ist es bekannt, Zellen an einer Pipette mit Unterdruck anzusaugen (vgl. US-Z "NATURE", Vol. 260, Seiten 799-801, 1976). Bei der Patch-Clamp-Technik muß jedoch die Pipette gezielt an eine einzelne Zelle herangeführt werden. Bei der Patch-Clamp-Technik werden die zu kontaktierenden Zellen nicht bewegt, da sie in der Regel an einem Substrat anhaften. Das herkömmliche Kontaktieren von Zellen mit Patch-Clamp-Pipetten hat jedoch den Nachteil, daß die Anzahl gleichzeitig kontaktierbarer Zellen äußerst beschränkt ist, da aus Platzgründen nicht beliebig viele Pipetten in die Kulturkammer eingeführt werden können.

Die Patch-Clamp-Technik hat andererseits gegenüber der weiter oben beschriebenen Technik, bei der lediglich von der Außenseite der Zelle her Messungen möglich sind, den Vorteil, daß das Zellinnere in die Messung mit einbezogen werden kann.

Bei der konventionell angewandten Patch-Clamp-Technik mit Einzelpipetten wird dies unter mikroskopischer Beobachtung dadurch bewirkt, dass eine fragile Glaspipette mittels eines Mikromanipulators an eine auf einem Substrat haftende einzelne Zelle herangeführt und die Membran vorsichtig an die Pipettenöffnung angesaugt wird. Es besteht daher ein direkter Kontakt zwischen Glas- und Membranoberfläche. Dadurch wird ein Membranfleck gegenüber der umgebenden Flüssigkeit abgedichtet und elektrisch isoliert. Diese Isolation wird auch als "Gigaseal" bezeichnet. Von dieser "Cell-Attached-Konfiguration" gelangt man zur so genannten "Whole-Cell-Konfiguration", indem man die abgedichtete Membran weiter angesaugt. Dies geschieht derart, dass das Membranstück unterhalb der Pipette durchbrochen wird. Auf diese Weise entsteht ein hydraulisch und elektrisch abgedichteter Zugang über die Pipettenöffnung zum Zellinneren. Die restliche Zellmembran ist damit als Ganzes elektrisch zugänglich (sogenannter "whole cell patch"). Die Anwendung dieser herkömmlichen Methode erfordert jedoch ein gehöriges Maß an Erfahrung und Fingerspitzengefühl. Mehrere Zellen können nur sequentiell bearbeitet werden. Diese Methode ist daher ungeeignet für Massenuntersuchungen, wie sie z.B. im Bereich des Pharmascreening, des Substanzscreening und dergleichen erforderlich wären.

Die nichtvorveröffentlichte WO 01/25769 beschreibt eine gattungsgemäße Vorrichtung, bei der in einem Substrat ein Kanal angeordnet ist, der über eine Öffnung mit einer Kavität in Verbindung steht. Beidseits der Öffnung sind Elektroden angeordnet, über die eine Zelle messtechnisch erfasst werden kann, die durch Unterdruck in dem Kanal auf die Öffnung gesogen wird. Eine Mehrzahl von Kanälen, über die Flüssigkeit zu- oder abführbar ist, ist aus dieser Druckschrift nicht bekannt.

Die Veröffentlichung Kostyuk et al: "Effect of internal fluoride and phosphate on membrane currents during intracellular dialysis of nerve cells", Nature 257, 23. Oktober 1975, S. 691, beschreibt ein Verfahren zur intrazellulären Dialyse und Spannungsmessung an Zellen, wobei die jeweilige Zelle nicht auf einer Öffnung in einem planaren Substrat positioniert wird, sondern in eine sich konisch verjüngende Öffnung in dem planaren, relativ dicken Substrat eingesogen wird.

Der Erfindung liegt daher die Aufgabe zugrunde, eine Vorrichtung der eingangs genannten Art dahingehend weiterzubilden, dass die vorstehend genannten Nachteile vermieden werden. Insbesondere soll die Erfindung möglichst konsistente Messungen vorzugsweise parallel an einer Mehrzahl von Zellen ermöglichen, insbesondere wie es im Bereich des experimentellen und anwendungsorientierten Screenings der Wirkung von pharmazeutischen Wirkstoffen auf zellulärer Ebene gewünscht wird.

Bei einer Vorrichtung gemäß der eingangs genannten Art wird die der Erfindung zugrunde liegende Aufgabe gelöst durch die Merkmale von Anspruch 1.

Die der Erfindung zugrunde liegende Aufgabe wird auf diese Weise vollkommen gelöst.

Bei der erfindungsgemäßen Vorrichtung ist die zweite Elektrode an dem von der Unterseite der Membran einer auf der Oberfläche des Substrates positionierten Zelle abgewandten Ende des Kanals angeordnet, wobei die erste und die zweite Elektrode zur elektrischen Abfrage der Zelle geeignet sind, die zweite Elektrode ist somit an dem von der ersten Elektrode abgewandten Ende des Kanals angeordnet.

Die Zelle wird also über eine Elektrode, die in Richtung des Kanals von der Unterseite der Membran beabstandet ist, elektrisch abgefragt. Hierzu kann ein Strom in das Zellinnere injiziert werden.

Dies hat den Vorteil, dass ein direkter elektrischer Zugang nur in das Innere der Zelle hergestellt wird. Dadurch, dass die Zelle mit ihrer Außenmembran dicht auf dem Boden der Mikroküvette aufliegt und dort sogar mittels Unterdruck fixiert wird, entsteht in automatisierter Weise der aus der herkömmlichen Patch-Clamp-Technik bekannte Gigaseal, d.h. ein extrem hoher und daher die Messung wenig beeinflussender Leckwiderstand zwischen Intrazellulär- und Extrazellulärmedium. Dadurch, dass die Elektrode sich räumlich entfernt vom Gigaseal befindet, ist ferner gewährleistet, dass die Zelle selbst nur mit elektrisch isolierenden Materialien in Verbindung kommt, so dass die Aufrechterhaltung des Gigaseals gewährleistet ist.

Die Erfindung ermöglicht es, im Vergleich zu herkömmlichen Patch-Clamp-Techniken, auf das diffizile Manipulieren einer fragilen Glaspipette zu verzichten, da die Funktion der herkömmlichen Glaspipette durch den Kanal in einem Substrat ausgeübt wird, an den ein Unterdruck angelegt wird, um eine Cell-Attached-Konfiguration einzustellen. Hat sich auf diese Weise ein Megaseal zwischen der Zellwand und der Oberfläche, an die die Zelle angesaugt wird, eingestellt, so wird erfindungsgemäß entweder die Unterseite der Membran durch eine Erhöhung des Unterdrucks aufgerissen, so dass nunmehr Messungen über den Kanal durch das von der Membran umschlossene Zellinnere durchgeführt werden können. Alternativ oder zusätzlich hierzu kann die Membran durch Zugabe von porenbildenden Substanzen mikroporös und elektrisch niederohmig gemacht werden. Durch die Zugabe solcher porenbildender Substanzen, wie etwa Nystatin oder Amphotericin B, werden in der Membran Poren gebildet, so dass ein niederohmiger Zugang zum Zellinnern ermöglicht wird, durch den jedoch keine größeren Moleküle diffundieren können.

Somit können die resultierenden Membranströme gemessen werden, ohne dass hierzu die Unterseite der Membran zerstört werden muss. Alternativ kann die Membran in diesem Bereich auch durch einen kurzzeitigen elektrischen Impuls durchlässig gemacht werden oder aufgerissen werden.

Ein besonderer Vorteil der Erfindung liegt darin, dass die Messelektrode räumlich entfernt von der Membran angebracht werden kann, so dass die Zelle nicht mit ihrer Membran unmittelbar auf der Elektrode aufliegt, sondern die Messung über das intrazelluläre Medium erfolgt.

Während bei der bekannten Anordnung gemäß der DE 197 12 309 A1 nur extrazelluläre Messungen möglich sind, d.h. Messungen von durch Membranströme hervorgerufenen Potentialänderungen in unmittelbarer Umgebung der Zelle, können erfindungsgemäß intrazelluläre und extrazelluläre Messungen durchgeführt werden, d.h. es kann die über die Membran hinweg anliegende Spannung gemessen und kontrolliert werden. Bevorzugt wird zu diesem Zweck ein Strom in die Membran injiziert.

Die Erfindung eignet sich daher in besonderem Maße für Massenuntersuchungen im Bereich des Pharmascreening und des Substanzscreening, der Identifizierung von Klonen (GVO's; genetisch veränderte Organismen) und im Rahmen von Substanzoptimierungen, wobei das Zytoplasma biologischer Zellen elektrisch gemessen wird, und zwar gleichzeitig oder unmittelbar sequentiell für eine Vielzahl derartiger Zellen. Die Erfindung eröffnet daher erstmalig die Möglichkeit, eine Technik mit denselben Vorteilen herkömmlicher Patch-Clamp-Techniken in vollautomatisierter Weise einzusetzen. Es können daher viele derartige Zellen parallel, automatisierbar und mit hohem Durchsatz untersucht werden.

Dass der Kanal an seinem der ersten Elektrode abgewandten Ende über Ventile mit einer Mehrzahl von Kanälen verbunden ist, über die Flüssigkeit zu- oder abführbar ist, hat den Vorteil, dass durch entsprechende Kontrolle der Druckverhältnisse in den Verbindungskanälen das Zellinnere nach Ausbildung der Whole-Cell-Konfiguration, d.h. nach Durchbrechen der Membran, mit Intrazellulär-Flüssigkeit in Kontakt kommt.

Dabei kann die Zusammensetzung des intrazellulären flüssigen Mediums nach dem Öffnen der Membran oder der Erzeugung von Mikroporen durch Zugabe von Substanzen verändert oder das intrazelluläre flüssige Medium ausgetauscht werden. Hierzu kann der Kanal mit zwei oder mehr getrennten Verbindungskanälen verbunden werden, wobei einer mit Elektrolyt gefüllt wird, der in seiner Zusammensetzung derjenigen des Zytoplasmas (Intrazellulärflüssigkeit) ähnelt oder eine spezielle Flüssigkeit mit Wirkstoffzusätzen ist.

Auf diese Weise wird eine gezielte und kontrollierte Beeinflussung des intrazellulären Mediums ermöglicht und gleichzeitig das mögliche Spektrum von durchführbaren Messungen erheblich vergrößert.

In bevorzugter Weiterbildung der erfindungsgemäßen Vorrichtung sind Mittel zur Steuerung des Druckgefälles vorgesehen, sowohl zur Erzeugung eines statischen Druckgefälles zur Einstellung einer Cell-Attached-Konfiguration, als auch zur pulsartigen Erhöhung des Druckgefälles zum Aufreißen der Unterseite der Membran.

Auf diese Weise kann einerseits die Einstellung des Megaseals auf zuverlässige und kontrollierte Weise erreicht werden und andererseits der Megaseal aufrechterhalten werden, während durch einen kurzen Druckimpuls die Unterseite der Membran aufgerissen wird und sich an die Kanalwandung anlegt. Dabei ist die Steuerung vorzugsweise so ausgelegt, dass der statische Unterdruck ständig aufrechterhalten wird (Offset-Druck), so dass der Megaseal auch bei der Whole-Attached-Konfiguration aufrechterhalten wird.

Die zweite Elektrode kann bei der neuen Vorrichtung das abgewandte Ende des Kanals ringförmig umgeben.

Diese Maßnahmen haben den Vorteil, dass die Elektrode in einfacher Weise in eine Mikrostruktur integriert werden kann, indem sie auf der Unterseite der Schicht ausgebildet wird, in der der Kanal verläuft.

Bei einer anderen Variante der Erfindung ist die zweite Elektrode hingegen im Abstand vom abgewandten Ende des Kanals angeordnet.

Diese Maßnahme hat den Vorteil, dass die Elektrode, wie noch zu erläutern sein wird, gegenüber dem Kanal auch beweglich angeordnet sein kann, so dass mit derselben Elektrode nacheinander mehrere Zellen vermessen werden können.

In zusätzlicher Weiterbildung der Erfindung ist über dem Substrat eine Mikroküvette angeordnet, in deren Boden eine Öffnung vorgesehen ist.

Auf diese Weise lässt sich die Flüssigkeit in einer insbesondere für Massenuntersuchung geeigneten Weise oberhalb des Substrates speichern, indem der Kanal oder die Kanäle vorgesehen sind. Hierbei können die Zellen durch eine geeignete trichterförmige Ausbildung der Mikroküvette unmittelbar bis in die Nähe einer Kanalmündung einer Substratoberfläche geführt werden. Auf alternative Weise kann die Öffnung im Boden der Mikroküvette jedoch auch einen deutlich größeren Durchmesser haben, so dass eine Führung der Zelle bis zur Mündung des Kanals im Wesentlichen durch den angelegten Unterdruck erreicht wird. Dies erleichtert die Herstellung der erfindungsgemäßen Struktur.

Ferner ist es bevorzugt, eine Vielzahl von Kanälen in einem gemeinsamen Substrat anzuordnen.

Hierdurch ist eine kompakte Bauweise bei einfacher Herstellung erreichbar.

Dabei sind ferner bevorzugt eine Vielzahl von Mikroküvetten in einer Platte angeordnet.

Hierdurch ergibt sich der Vorteil, daß parallele oder sequentielle Messungen an vielen Zellen in einfacher Weise vorbereitet werden können, weil sich alle Mikroküvetten in einer gemeinsamen Platte befinden.

Bei Ausführungsformen der Erfindung, die eine gemeinsame Platte für die Mikroküvetten verwenden, ist weiterhin bevorzugt, wenn die Platte mehrschichtig aufgebaut ist.

Diese Maßnahme hat den Vorteil, den unterschiedlichen Anforderungen an die verschiedenen Elemente der Platte durch geeignete Werkstoffwahl Rechnung tragen zu können.

Dies gilt insbesondere dann, wenn bei einer Fortbildung dieser Variante die Platte eine obere Schicht, eine mittlere Schicht und eine Unterschicht umfaßt, wobei in der oberen Schicht die Mikroküvetten angeordnet sind, die mittlere Schicht das Substrat mit den Kanälen bildet und in der Unterschicht Verbindungskanäle, die zu den Kanälen führen, angeordnet sind, sowie in einer bevorzugten Ausführungsform elektrische Zuleitungen, die zu den Kanälen führen, und Mikroelektroden angeordnet sind.

Diese Dreiteilung der Platte hat den Vorteil, daß für die drei wesentlichen Funktionen jeweils einzelne Schichten unterschiedlicher Dicke und unterschiedlicher Materialien eingesetzt werden können.

Gemäß einer weiteren Ausführung der Erfindung ist das Substrat mit einer Unterschicht verbunden, die aus einer oder mehreren Schichten aus photostrukturierbaren Materialien besteht, die eine räumliche Führung von Verbindungskanälen erlauben, die zu den Kanälen führen.

Hierbei kann die Unterschicht auf einen Glasträger aufgebracht sein.

Durch diese Maßnahmen wird eine besonders kompakte Bauweise und eine einfache Herstellung ermöglicht. Als photostrukturierbare Materialien sind bestimmte Polymere, aber auch bestimmte Gläser bekannt.

Es ist bevorzugt, wenn die Verbindungskanäle eine Breite zwischen 10 $\mu$m und 40 $\mu$m, vorzugsweise etwa 20 $\mu$m aufweisen.

Die Kanäle selbst haben bevorzugt eine lichte Weite von weniger als 10 $\mu$m, vorzugsweise von weniger als 5 $\mu$m.

Diese Maße haben sich im vorliegenden Zusammenhang als optimal erwiesen. Insbesondere wird dadurch, daß die lichte Weite der Kanäle geringer als der Zelldurchmesser ist, die Positionierung je einer Zelle auf einem Kanal unterstützt.

Wie bereits weiter oben angedeutet wurde, ist bei diesen Ausführungsformen der Erfindung besonders bevorzugt, wenn die Elektroden auf der Unterseite der mittleren Schicht oder der Oberseite der Unterschicht angeordnet sind.

Diese Maßnahme hat den Vorteil, daß die Elektroden samt ihren Zuleitungen durch einfaches Aufdrukken, Abscheiden, Aufdampfen und nachfolgende Mikrostrukturierung durch bekannte Verfahren (Photolitographie, Ätzverfahren, lift-off etc.) ausgebildet werden können.

Es ist in diesem Fall weiter bevorzugt, wenn die Elektroden in Draufsicht als quadratische Fläche mit einer Kantenlänge zwischen 20 $\mu$m und 60 $\mu$m, vorzugs-

weise etwa 40 μm ausgebildet sind.

Wie bereits erwähnt, kann in diesem Fall vorgesehen sein, die zu den Elektroden führenden Leiterbahnen zwischen der mittleren und der unteren Schicht anzuordnen. Dies kann alternativ dadurch geschehen, daß sie unten auf die mittlere oder oben auf die Unterschicht aufgetragen werden. Ein Auftrag auf die Unterseite der mittleren Schicht hat den Vorteil, daß die Leiterbahnen zusammen mit den Elektroden ausgebildet werden können, insbesondere auch mit demselben Werkstoff, insbesondere Edelmetall, vorzugsweise Gold.

Die Leiterbahnen haben dabei vorzugsweise eine Breite zwischen 5 μm und 30 μm, insbesondere etwa 10 μm.

Wie bereits erwähnt wurde, können bei einer mehrschichtigen Bauweise der Platte unterschiedliche Werkstoffe für die einzelnen Schichten verwendet werden.

Die verschiedenen Schichten können z.B. unabhängig voneinander aus Kunststoff, aus Polymethylmethacrylat (PMMA), Silikon, PTFE, Polyimid oder aus einem anorganischen Material, insbesondere aus Glas, Keramik oder Silizium hergestellt werden.

Für das Substrat wird vorzugsweise Polyimid verwendet, das zu diesem Zweck als Folie eingesetzt wird, in der die Kanäle als Bohrungen ausgebildet sind. Das Substrat (die Folie) hat dann vorzugsweise eine Dicke zwischen 2 μm und 40 μm, vorzugsweise etwa 5 μm.

Für die untere Schicht wird vorzugsweise Glas verwendet. Im Glas, das in nahezu beliebiger Dicke zur Verfügung gestellt werden kann, um auf diese Weise auch die mechanische Stabilität sicherzustellen, können die notwendigen Verbindungskanäle und dergleichen in herkömmlicher Weise ausgebildet werden.

In zusätzlicher Weiterbildung der Erfindung ist es bevorzugt, das Substrat an der Unterseite einer Platte anzuordnen, in der eine Mehrzahl von Bohrungen als Mikroküvetten ausgebildet ist, in deren Boden Löcher vorgesehen sind, mit denen die Kanäle des Substrates zentriert sind.

Auf diese Weise läßt sich ein kombinierter Körper mit einer Vielzahl von Mikroküvetten, denen einzelne Kanäle und Elektroden zugeordnet sind, auf relativ einfache Weise herstellen.

Gemäß einer weiteren Ausführung der Erfindung ist eine Hydraulik- und Meßeinheit vorgesehen, die eine zur Unterseite des Substrates hin offene Kammer aufweist, die an der Unterseite des Substrates derart positionierbar ist, daß die Kammer mit einem ausgewählten Kanal kommuniziert und nach außen abgedichtet ist, wobei die Kammer mindestens eine Elektrode enthält und mit mindestens einem Anschlußkanal verbindbar ist, der mit einer Unterdruckquelle verbunden ist.

Dabei kann ferner eine Verfahreinheit zum Verfahren und Positionieren der Platte und der Unterdruck- und Meßeinheit relativ zueinander vorgesehen sein.

Auf diese Weise kann eine einzige Meßeinheit zur sequentiellen Vermessung einer Vielzahl von Zellen verwendet werden, wodurch sich eine erhebliche Kostenersparnis ergeben kann.

Dabei können bevorzugt handelsübliche, herkömmliche Normrasterplatten (sogenannte "96-Loch-Platten", "384-Loch-Platten" oder ähnliche) verwendet werden. Diese brauchen lediglich nach unten hin durch das Aufbringen der mit Kanälen (Löchern) versehenen Folie abgeschlossen zu werden. Die Messungen an den vielen einzelnen Zellen in den Bohrungen der Lochplatte werden nachfolgend sequentiell durch Verfahren der Unterdruck- und Meßeinheit oder umgekehrt durch Verfahren der Normrasterplatten in bezug auf eine feststehende Unterdruck- und Meßeinheit durchgeführt. Diese erzeugt den Unterdruckimpuls zum Öffnen der Zelle und enthält auch die Elektrode, um die anschließende Messung durch das Zellinnere durchzuführen.

Hierbei kann wiederum, wie zuvor bereits erwähnt, die Kammer mit einer Mehrzahl von Anschlußkanälen über Ventile verbunden sein, um so das Zellinnere nach Ausbildung der Whole-Cell-Konfiguration mit Intrazellulärflüssigkeit in Kontakt zu bringen oder die Zusammensetzung der Intrazellulärflüssigkeit verändern zu können.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:

Fig. 1    eine äußerst schematisierte perspektivische Ansicht eines Ausführungsbeispiels einer erfindungsgemäßen Vorrichtung;

Fig. 2    einen Schnitt durch eine Mikroküvette der Anordnung gemäß Fig. 1, ebenfalls stark schematisiert;

Fig. 3    eine Draufsicht auf die Mikroküvette gemäß Fig. 2, in leicht verkleinertem Maßstab;

Fig. 4    einen Ausschnitt aus Fig. 2, in weiter vergrößertem Maßstab, zur Erläuterung des erfindungsgemäßen Verfahrens;

Fig. 5    eine Darstellung, ähnlich Fig. 3, darstellend den Stand der Technik;

Fig. 6    eine perspektivische Ansicht sowie einen vergrößerten Ausschnitt daraus, eines Vergleichs beispiels; und

Fig. 7a) bis 7c)  verschiedene Phasen beim Ansaugen einer Zelle, der Ausbildung einer Cell-Attached-Konfiguration und einer Whole-Cell-Konfiguration bei Verwendung von zwei Verbindungskanälen zu dem Kanal, in vereinfachter, schematischer Darstellung.

Beispiel 1

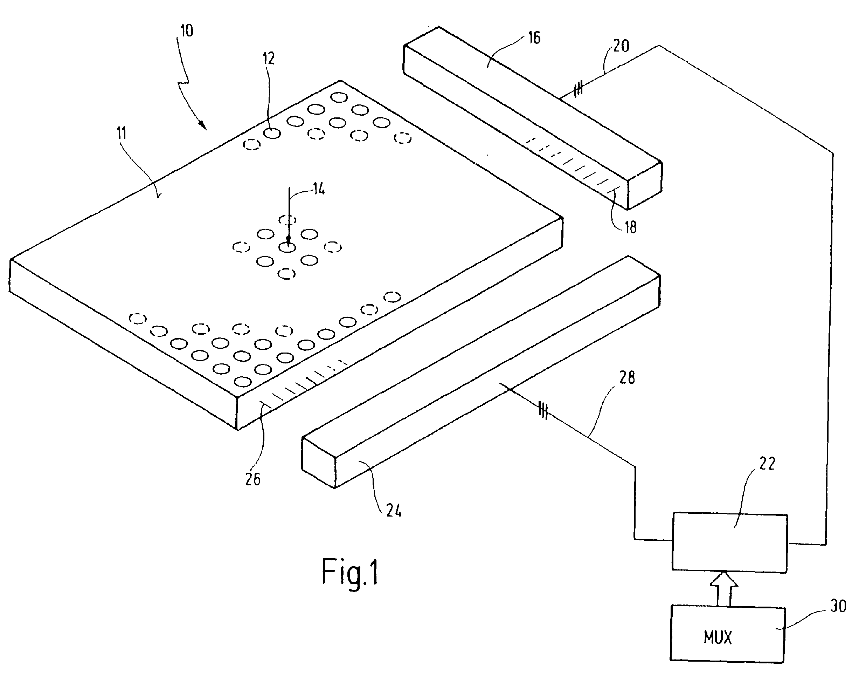

In den Figuren 1 bis 4 ist mit 10 eine Platte bezeichnet. In einer Oberfläche 11 der Platte 10 ist durch Abformen ein Raster von Mikroküvetten 12 ausgebildet. Die Mikroküvetten 12 sind dreidimensional und haben für die Kultur von Zellen geeignete Abmessungen. In einer Platte 10 können z.B. 8 x 12 = 96 Mikroküvetten 12 angeordnet sein.

Mit einem Pfeil 14 ist angedeutet, daß die Mikroküvetten 12 von oben befüllt werden können, und zwar mit einer Flüssigkeit, in der sich zu untersuchende Zellen befinden. Es ist dabei möglich, die Mikroküvetten 12 jeweils individuell mit unterschiedlichen Flüssigkeiten und Zellen zu befüllen.

Zum Ausführen der Messungen ist ein elektrisches Anschlußmodul 16 vorgesehen, das seitlich an die Platte 10 angedockt werden kann, wozu eine ausreichende Vielzahl von Steckern 18 vorgesehen ist. Die Stecker 18 stehen mit einem Netz von Leiterbahnen in Verbindung. Diese Leiterbahnen führen zu Elektroden, die im Bereich der Mikroküvetten 12 angebracht sind, wie dies noch erläutert werden wird. Vom elektrischen Anschlußmodul führt eine Datenleitung 20 zu einem Steuergerät 22.

Weiterhin ist ein hydraulisches Anschlußmodul 24 vorgesehen, das ebenfalls seitlich an die Platte 10 angedockt werden kann, und zwar mittels einer entsprechenden Vielzahl hydraulischer Stecker 26. Über das hydraulische Anschlußmodul 24 kann in vorbestimmter Weise, insbesondere individuell, unterhalb der Mikroküvetten 12 ein Unterdruck erzeugt werden, insbesondere mit gepulstem zeitlichem Verlauf, wie dies weiter unten noch ausführlich erläutert werden wird.

Zu diesem Zweck sind die hydraulischen Stecker 26 über ein Netzwerk von Verbindungskanälen und Öffnungen 49 am Boden der Mikroküvetten mit den Mikroküvetten 12 verbunden. Wenn alle Mikroküvetten 12 mit demselben Unterdruck beaufschlagt werden sollen, sind alle Verbindungskanäle parallel geschaltet und werden im hydraulischen Anschlußmodul 24 mit einer zentralen, gesteuerten Unterdruckquelle direkt verbunden. Wenn jedoch die einzelnen Mikroküvetten 12 mit jeweils individuellem Unterdruck angesteuert werden sollen, kann ebenfalls eine zentrale Unterdruckquelle verwendet werden, die an das Netzwerk von Verbindungskanälen angeschlossen ist, wobei sich dann in diesen Verbindungskanälen individuell steuerbare Ventile befinden. Alternativ können aber in den Verbindungskanälen auch miniaturisierte Pumpen, insbesondere Miniatur-Membranpumpen, angeordnet sein, die individuell angesteuert werden. Die elektrische Ansteuerung der Ventile und/ oder Miniaturpumpen kann entweder über das elektrische Anschlußmodul 16 oder das hydraulische Anschlußmodul 24 erfolgen. In jedem Fall führt eine Leitung 28 zum Ansteuern der vorerwähnten Elemente vom hydraulischen Anschlußmodul 24 zum Steuergerät 22.

Das Steuergerät 22 steht seinerseits mit einem Multiplexer 30 in Verbindung, um in vorbestimmter Weise mehrere Messungen gleichzeitig oder gegebenenfalls sequentiell durchführen zu können.

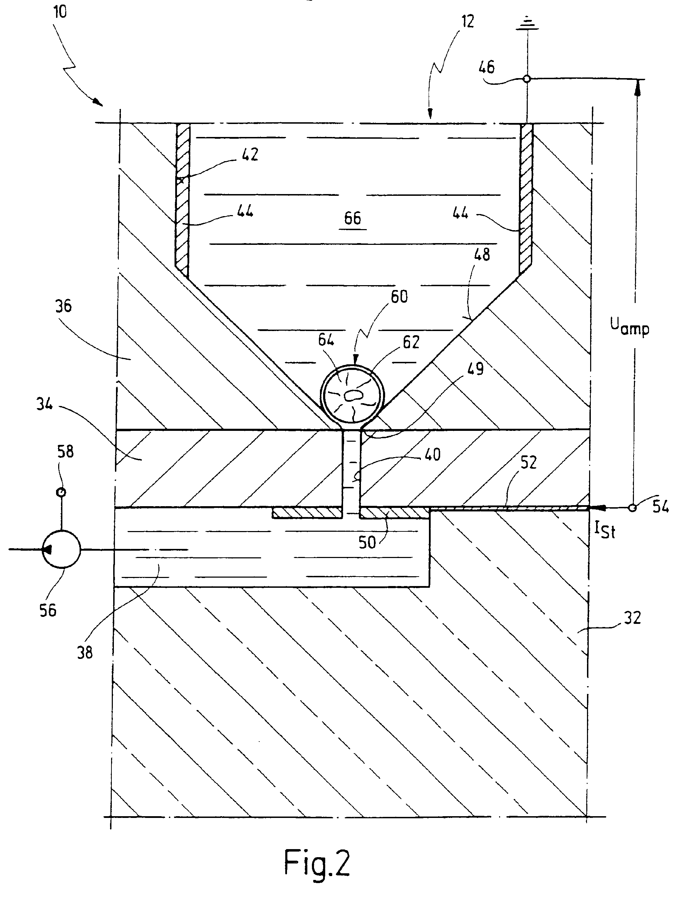

Wie man aus Fig. 2 erkennt, besteht die Platte 10 im wesentlichen aus drei Schichten. Auf der Unterschicht 32 befindet sich eine mittlere Schicht oder ein Substrat 34, das als Folie ausgebildet ist. Eine obere Schicht 36 ist als Mikrostrukturschicht ausgeführt. Die Unterschicht 32 besteht dabei vorzugsweise aus Glas. Das Substrat 34 ist vorzugsweise eine Polyimidfolie. Die Mikrostrukturschicht 36 besteht demgegenüber vorzugsweise aus Polymethylmethacrylat (PMMA).

In der Oberseite der Unterschicht 32 ist ein Verbindungskanal 38 ausgeführt. Der Verbindungskanal 38 dient zum individuellen Ansteuern der in Fig. 2 dargestellten Mikroküvette 12. Der Verbindungskanal 38 steht über einen vertikalen Kanal 40 im Substrat 34 mit einer Öffnung 49 am Boden 48 der Mikroküvette 12 in Verbindung. Die Mikroküvette 12 ist an ihrer Oberseite mit einem zylindrischen Abschnitt 42 versehen, der mit einer Referenzelektrode 44 ausgekleidet ist. Die Referenzelektrode 44 ist an einen ersten elektrischen Anschluß 46 angeschlossen. Dieser liegt vorzugsweise auf Masse.

Unten an den zylindrischen Abschnitt 42 schließt sich ein trichterförmiger Abschnitt an, der den Boden 48 bildet, in dem die Öffnung 49 vorgesehen ist.

Eine Elektrode 50 ist näherungsweise ringförmig um das untere Ende des vertikalen Kanals 40 herum angeordnet. Sie ist zu diesem Zweck auf die Unterseite des Substrates 34 aufgebracht. Die Elektrode 50 ist an eine Zuleitung 52 angeschlossen, die zwischen Unterschicht 32 und dem Substrat 34 verläuft. Die Zuleitung 52 kann z.B. zusammen mit der Elektrode 50 auf die Unterseite des Substrates 34 aufgedruckt, aufgedampft, abgeschieden oder dergleichen sein. Die Zuleitung 52 ist mit einem zweiten elektrischen Anschluß 54 verbunden.

Die Elektroden 46 und 50 bestehen aus Silber/ Silberchlorid (Ag/AgCl). Derartige Elektroden werden in der Fachwelt als "reversibel" oder als "nicht polarisierbar" bezeichnet. Sie haben den Vorteil, daß an den Zellen nicht nur Wechselspannungsmessungen, also Messungen der Potentialspitzen (sog. "spikes"), sondern auch Gleichspannungsmessungen möglich sind. Sie können auch zur Strominjektion verwendet werden.

Zwischen den Elektroden 46 und 50 wird die Meßspannung $U_{amp}$ gemessen. Ferner kann über den zweiten Anschluß 54 parallel zur Spannungsmessung ein Stimulationsstrom $I_{st}$ eingespeist werden.

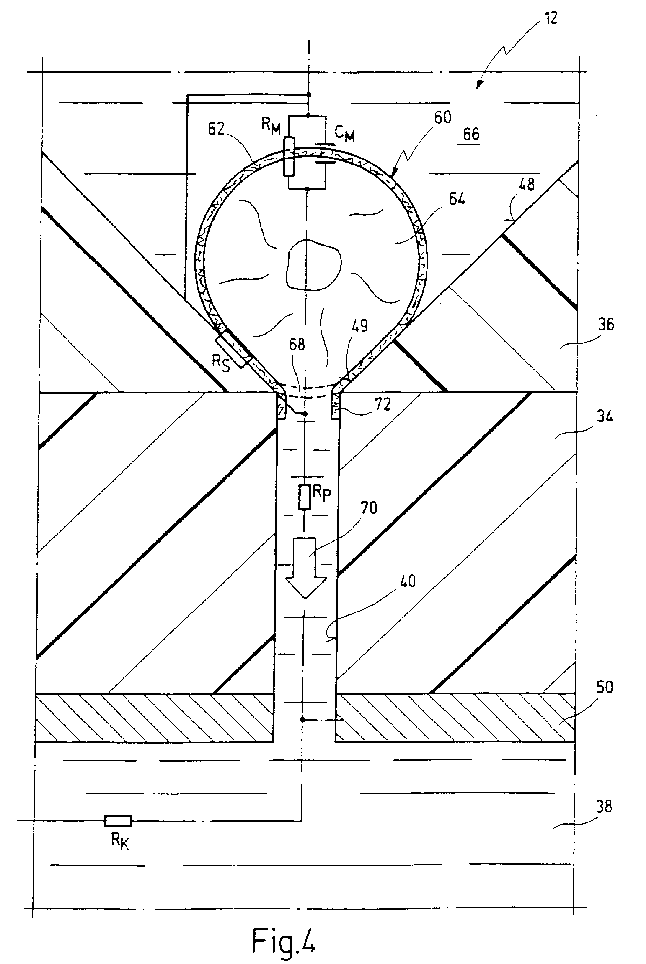

Dies wird weiter unten anhand von Fig. 4 noch näher erläutert werden.

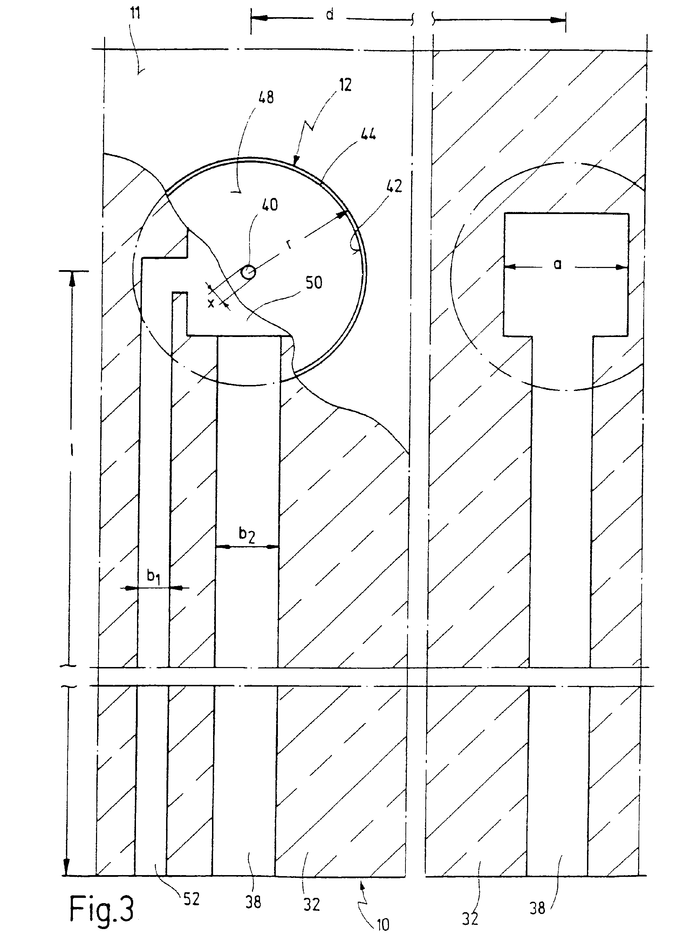

Wie man aus der Draufsicht gemäß Fig. 3 erkennen kann, sind mehrere Elektroden 12 in der Platte 10 in Form eines Rasters angeordnet, wobei das Rastermaß d zwischen 0,1 und 10 mm, vorzugsweise bei etwa 9 mm liegt.

Die Mikroküvetten 12 haben im Bereich ihres zylindrischen Abschnitts 42 einen Innenradius r zwischen etwa 1 und 9 mm, vorzugsweise von etwa 7 mm, was eine leichte Befüllung erlaubt. Die lichte Weite x des vertikalen Kanals 40 beträgt weniger als 10 $\mu$m, vorzugsweise weniger als 5 $\mu$m.

Die Leiterbahnen 52 haben eine Breite $b_1$ zwischen 5 $\mu$m und 30 $\mu$m, vorzugsweise etwa 10 $\mu$m. Die Elektroden 50 sind in Draufsicht vorzugsweise quadratisch ausgebildet und haben eine Kantenlänge a zwischen 20 $\mu$m und 60 $\mu$m, vorzugsweise etwa 40 $\mu$m. Die Verbindungskanäle 38 haben einen Breite $b_2$ zwischen 10 $\mu$m und 40 $\mu$m, vorzugsweise etwa 20 $\mu$m.

Das Substrat 34 bzw. die Folie ist zwischen 2 $\mu$m und 40 $\mu$m, vorzugsweise etwa 5 $\mu$m dick.

Der Abstand 1 der Mikroküvetten 12 vom Rand der Platte 10 beträgt vorzugsweise mindestens 2 cm, wodurch ein hoher Shuntwiderstand, d.h. eine elektrische Entkopplung der einzelnen Elektroden erreicht wird.

Die Elektrode 50 und die Leiterbahnen 52 bestehen vorzugsweise aus Gold.

In Fig. 2 ist noch angedeutet, daß in den Verbindungskanal 38 eine Mikropumpe 56 integriert sein kann, die mittels eines dritten Anschlusses 58 ansteuerbar ist. Mittels der Mikropumpe 56 oder mittels einer zentralen Unterdruckquelle, gegebenenfalls unter Einschaltung von Ventilen in den Verbindungskanälen 38, kann ein Unterdruck mit gezieltem zeitlichem Verlauf in den Verbindungskanälen 38 eingestellt werden.

Fig. 4 zeigt in weiter vergrößertem Maßstab die Situation, wenn sich eine Zelle 60 auf dem Boden 48 der Mikroküvette 12 abgesenkt hat. Dies geschieht entweder durch Schwerkraft oder gezielt dadurch, daß durch Anlegen eines vorzugsweise konstanten Unterdrucks im Kanal 40 die Zelle 60 gezielt angesaugt wird. Die trichterförmige Gestalt des Abschnittes am Boden 48 der Mikroküvette 12 bewirkt darüber hinaus eine Vereinzelung der Zellen, so daß im Regelfall nur eine einzige Zelle 60 auf der Öffnung 49 am Boden 48 der Mikroküvette 12 über dem Kanal 40 liegen wird.

In Fig. 4 ist die Außenhaut oder Membran der Zelle 60 mit 62 und das Zellinnere mit 64 bezeichnet. Die Zelle 60 befindet sich in einer umgebenden Flüssigkeit 66, die in dem gezeigten Ausführungsbeispiel auch die Verbindungskanäle 38 und die Kanäle 40 ausfüllen kann und dort austauschbar ist. Sie liegt mit ihrer Unterseite 68 auf dem Boden 49 auf.

Sobald diese Position erreicht ist, wird im Verbindungskanal 38 ein Unterdruckimpuls erzeugt, wie in Fig. 4 mit einem Pfeil 70 angedeutet. Dieser Unterdruckimpuls 70 ist so bemessen, daß die Unterseite 68 aufgerissen und nach Art eines Kragens 72 in den Kanal 40 hineingestülpt wird. Das Zellinnere 64 steht dann direkt mit dem Kanal 40 bzw. der darin befindlichen Flüssigkeit in Verbindung. Alternativ kann auch auf einen Unterdruckimpuls verzichtet werden und die Unterseite der Membran durch Zugabe von porenbildenden Substanzen, z.B. Nystatin oder Amphotericin B, durchlässig gemacht werden, so daß ein niederohmiger Zugang zum Zellinnern entsteht, jedoch keine größeren Moleküle hindurch diffundieren können.

In Fig. 4 ist ferner das elektrische Ersatzschaltbild der Zelle 60 eingezeichnet.

Mit $R_M$ und $C_M$ sind der Widerstand und die Kapazität der Membran 62 bezeichnet. $R_s$ ist der Sealwiderstand, d.h. der Isolationswiderstand zwischen Zellinnerem 64 und Extrazellulärmedium 66 außerhalb der Zelle 60. $R_p$ ist der Widerstand zwischen dem Zellinneren 64 und der Elektrode 50, während $R_k$ der Widerstand zwischen Elektrode 50 und Referenzelektrode 44 ist.

Dadurch, daß der Kragen 72 sich in den Kanal 40 hineinstülpt und an der Wand 48 anliegt, ohne jedoch die relativ weit entfernte Elektrode 50 zu erreichen, hat $R_s$ einen sehr hohen Wert ("Gigaseal").

Im stromlosen Zustand, wenn also über den Anschluß 54 und durch den Verbindungskanal 38 kein Strom fließt, entspricht die Spannung $U_{amp}$ der an der Zellmembran anliegenden Membranspannung $U_M$. Kann hingegen über den Verbindungskanal ein endlicher Strom fließen, gilt die Beziehung:

$$U_{amp} \;=\; \frac{R_k}{R_k \,+\, R_p}\, U_M\,,$$

d.h. die Membranspannung $U_M$ ist proportional zur Spannung $U_{amp}$.

Im Falle des Einleitens eines Stimulationsstromes $I_{ST}$ in den zweiten Anschluß 54 gilt für die Membranspannung $U_M$ im stationären Zustand die Beziehung:

$$U_M = \frac{R_s \cdot R_M}{R_M + R_s} I_{ST}\,,$$

für den Fall, daß $R_k \gg R_p + R_s R_m/(R_s + R_m)$ ist. Diese Bedingung wird erfindungsgemäß durch ausreichend lange Verbindungskanäle mit geringem Kanalquerschnitt erreicht.

Auf die vorstehend beschriebene Weise wird also die Zelle 60 am Boden 48 der Mikroküvette 12 sowohl hydraulisch als auch elektrisch kontaktiert, indem über die Elektrolytlösung der erforderliche Unterdruck und - durch zusätzliches Erzeugen eines Unterdruckimpulses 70 - der Öffnungsvorgang an der Zelle 60 bewirkt wird.

Vergleichsbeispiel

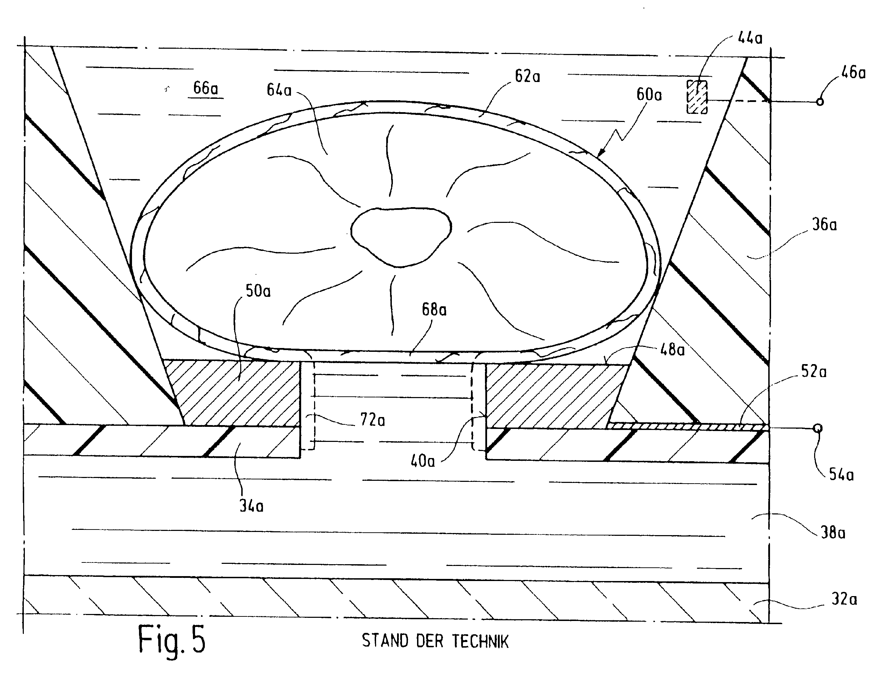

Um den Unterschied der erfindungsgemäßen Vorgehensweise zum Stand der Technik gemäß DE 197 12 309 A1 zu illustrieren, ist in Fig. 5 eine Darstellung ähnlich Fig. 4 gezeigt, die der bekannten Anordnung entspricht. Gleiche Elemente sind dabei mit gleichen Bezugszeichen versehen, wobei jeweils ein "a" hinzugefügt wurde.

Wie man aus Fig. 5 deutlich erkennt, liegt bei diesem Stand der Technik die Zelle 60a unmittelbar auf der Elektrode 50a auf. Die Zelle 60a wird daher von außen, d.h. von der Außenseite der Membran 62a, elektrisch beaufschlagt. Der Kanal 40a dient bei diesem Stand der Technik ausschließlich dazu, durch Anlegen eines gewissen, geringen Unterdrucks die Zelle 60a an den Boden 48a anzuziehen und dort zu fixieren, wobei der Boden 48a im Gegensatz zur vorliegenden Erfindung bei diesem Stand der Technik durch die Elektrode 50a gebildet wird.

Selbst wenn man bei diesem Stand der Technik über den Kanal 40a einen Unterdruckimpuls an die Zelle 60a legen und die Membran 62a öffnen würde (wovon in diesem Stand der Technik keine Rede ist), so würde sich ein Kragen 72a auf der Unterseite 68a der Zelle 60a bilden, der die Elektrode 50a im Bereich des Kanals 40a überdeckt. Trotz des Öffnens der Zelle 60a würde die Elektrode 50a somit immer noch keine direkte Messung durch das Zellinnere 64a ermöglichen, sondern auch weiterhin nur an der Außenseite der Membran 62a anliegen. Es würden daher die Messungen bei dieser Vorrichtung nach dem Stand der Technik auch bei einem Öffnen der Unterseite 68a der Zelle 60a nicht anders ablaufen als dort für den Fall beschrieben, bei dem der Unterdruck nicht zu einem Öffnen der Zelle 60a führt.

Weiteres Vergleichsbeispiel

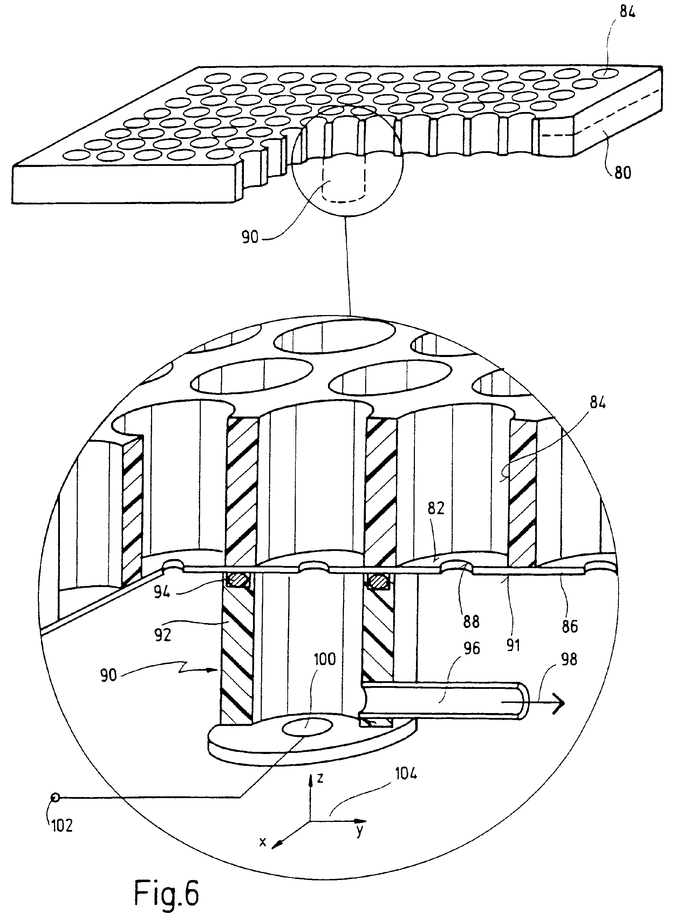

Schließlich zeigt Fig. 6 noch ein weiteres Vergleichsbeispiel.

Mit 80 ist in Fig. 6 eine Platte bezeichnet, die von an sich herkömmlicher Bauart ist. Platten 80 dieser Art werden als "96-Loch-Platte" bezeichnet. Sie enthalten 96 im Raster angeordnete vertikale zylindrische Bohrungen 84. Diese Bohrungen 84 können als Mikroküvetten eingesetzt werden. Die Platte 80 kann, wie in Fig. 6 rechts oben durch eine gestrichelte Linie angedeutet, auch mehrschichtig, insbesondere zweischichtig, aufgebaut sein.

Ein Boden 82 der zylindrischen Bohrungen oder Mikroküvetten 84 wird durch ein als Folie ausgebildetes Substrat 86 gebildet, das unten auf die Platte 80 geklebt, geschweißt oder sonstwie gebondet ist. Das Substrat 86 enthält jeweils im Zentrum des Bodens 82 einen Kanal 88, der als Loch ausgebildet ist.

Im Gegensatz zum erfindungsgemäßen Ausführungsbeispiel gemäß den Figuren 2 bis 4 befindet sich unterhalb des Substrates 86 kein Träger mit einem System von Verbindungskanälen. Statt dessen ist eine verfahrbare Hydraulik- und Meßeinheit 90 vorgesehen, die individuell von unten an die Unterseite 91 der Folie 86 herangeführt werden kann.

Die Einheit 90 umfaßt eine topfförmige Kammer 92, die an ihrer oberen Stirnseite mit einer Ringdichtung 94 versehen ist. Auf diese Weise kann die Kammer 92 dicht an die Unterseite 91 angesetzt werden, und zwar derart, daß die Vertikalachse der Kammer 92 mit der Achse jeweils eines Lochs 88 fluchtet.

Von der Kammer 92 führt eine Rohrleitung 96 zu einer nicht dargestellten Unterdruckeinheit. Auf diese Weise kann in der Kammer 92 ein Unterdruckimpuls erzeugt werden, wie mit einem Pfeil 98 angedeutet.

Am Boden der Kammer 92 ist eine Elektrode 100 angeordnet, die mit einem externen Anschluß 102 verbunden ist.

Mit 104 ist eine mehrachsige Verfahreinheit angedeutet. Die Verfahreinheit 104 gestattet es, die Hydraulik- und Meßeinheit 90 an der Unterseite 91 entlang zu führen, und zwar von Mikroküvette 84 zu Mikroküvette 84, um jeweils die Einheit 90 dann von unten dicht um das jeweilige Loch 88 herum an die Unterseite 91 anzupressen. Durch Beaufschlagen der Rohrleitung 96 mit einem Unterdruckimpuls 98 kann dann das gleiche Experiment durchgeführt werden, wie es weiter oben anhand Fig. 4 zum ersten Ausführungsbeispiel der Erfindung erläutert wurde.

Das erste Ausführungsbeispiel der Erfindung gemäß den Figuren 2 bis 4 hat den Vorteil, daß eine kompakte Platte mit sämtlichen Verbindungskanälen zum Ansteuern der Mikroküvetten 12 zur Verfügung steht, so daß ohne weitere Betätigungseinrichtungen, lediglich durch Ansteuern von Ventilen, Kontakten und dergleichen eine Vielzahl von Messungen sequentiell oder im Multiplex-Betrieb durchgeführt werden kann.

Das zweite Vergleichsbeispiel gemäß Fig. 6 hat demgegenüber den Vorteil, daß eine handelsübliche Platte eingesetzt werden kann und daß der Aufwand für eine Unterschicht mit einer Vielzahl von Verbindungskanälen, Leiterbahnen und einzelnen Elektroden gespart wird.

Beispiel 2

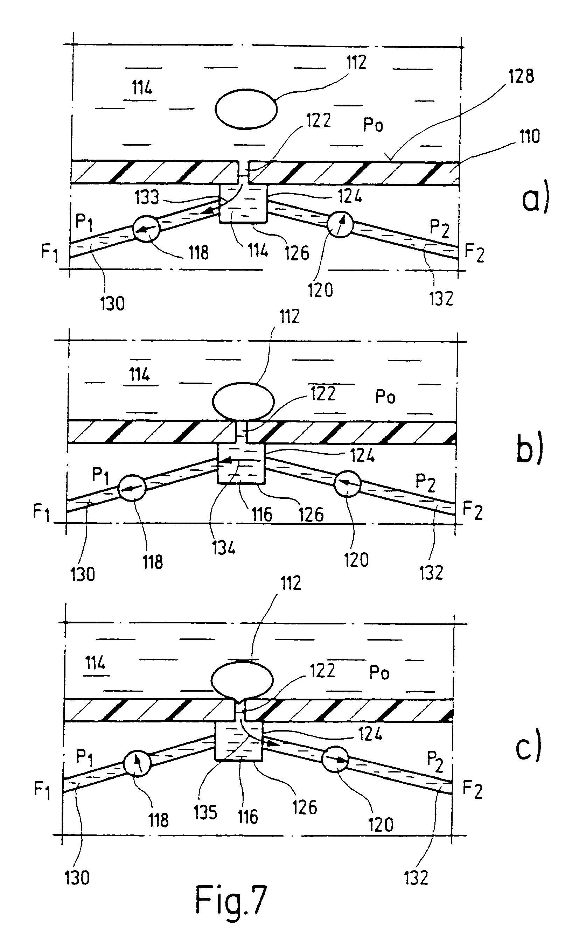

In den Fig. 7a) bis c) ist ein weiteres Ausführungsbeispiel der Erfindung äußerst schematisch dargestellt, das im folgenden näher erläutert wird.

Wiederum ist ein Substrat 110 vorgesehen, das beispielsweise aus einer Polyimid-Folie bestehen kann. In dem Substrat 110 ist eine Mehrzahl von Kanälen ausgebildet, von denen einer, der mit der Ziffer 122 bezeichnet ist, dargestellt ist. Oberhalb des Substrates 110 befindet sich eine Flüssigkeit, in der Zellen 112 vorhanden sind. Unterhalb des Kanals 122 ist eine Kammer 124 gebildet, die mit dem Kanal 122 kommuniziert und an deren Boden ähnlich wie bei der Ausführung gemäß Fig. 6 eine Elektrode 126 vorgesehen ist.

Im Unterschied zu den zuvor beschriebenen Ausführungen steht diese Kammer 124 jedoch nicht nur mit einem Verbindungskanal, sondern mit zwei Verbindungskanälen 130, 132, in Verbindung. Diese Verbindungskanäle 130, 132 sind über Ventile 118, 120 mit Flüssigkeitsreservoirs $F_1$ und $F_2$ verbindbar.

Es versteht sich, daß die Darstellung rein schematisch ist und daß die Verbindungskanäle 130, 132 z.B. in einer photopolimerisierbaren Schicht ausgebildet sein können und daß die Ventile vorzugsweise an den äußeren Enden der Kanäle ausgebildet sind.

Ist nun, wie in Fig. 7a) dargestellt, das Ventil 120 geschlossen und das Ventil 118 geöffnet, so führt das Anlegen eines Druckes $P_1$ an den Kanal 130, der geringer ist als der Druck $P_0$ in der Flüssigkeit 114 zur Ausbildung einer Strömung in Richtung des Pfeiles 133 durch den Kanal 122 und den Zuführkanal 130. Dies führt dazu, daß die Zelle 112 angesaugt wird und sich auf die Oberfläche 128 des Substrates 110 oberhalb der Öffnung des Kanales 122 aufsetzt und bei Aufrechterhaltung des Unterdruckes ein Megaseal ausbildet, so daß sich die Cell-Attached-Konfiguration einstellt.

Wird nunmehr gemäß Fig. 7b) das Ventil 120 geöffnet, wobei die Beziehung $P_1 < P_2 < P_0$ eingehalten wird, so füllt sich die Kammer 124 mit intrazellulärem Medium aus dem Flüssigkeitsreservoir $F_2$, während sich eine Strömung in Richtung des Pfeiles 134 aus dem Verbindungskanal 132 durch die Kammer 124 in den Zuführkanal 130 einstellt. Hierbei muß der Druck $P_2$ größer als der Druck $P_1$ sein, damit die Strömung in Richtung des Pfeiles 134 aus dem Verbindungskanal 132 zum Verbindungskanal 130 gerichtet ist; ferner müssen beide Drükke $P_1$ und $P_2$ geringer als der Druck $P_0$ in dem Extrazellulärmedium 114 sein, von dem die Zelle 112 umgeben ist.

Nunmehr wird in der nachfolgenden Phase gemäß Fig. 7c) das Ventil 118 geschlossen und ein pulsartiger Unterdruck an den Verbindungskanal 132 angelegt, so daß $P_2$ sehr viel kleiner als $P_0$ wird ($P_2 << P_0$). Dadurch wird die Unterseite der Membran der Zelle 112, der Membranpatch, angesaugt und infolge der Unterdruckstöße aufgebrochen, so daß sich nunmehr ein Whole-Cell-Patch-Clamp einstellt. Die Strömung erfolgt während dieser Phase in Richtung des Pfeiles 135 durch den Kanal 122 und den Verbindungskanal 132 zum Flüssigkeitsreservoir $F_2$ hin. Es wird nunmehr ein Zustand erreicht, in dem die Kammer 124 ausschließlich mit Intrazellulärmedium 116 gefüllt ist. Anschließend kann über das Ventil 118 wieder mit einem anderen Medium gearbeitet werden, sofern dies für die durchzuführenden Messungen erwünscht ist.

Der Vorteil dieser Anordnung und dieses Verfahrens liegt darin, daß mit genau kontrolliertem Intrazellulärmedium gearbeitet werden kann, dessen Zusammensetzung beeinflußt werden kann oder daß sogar ein anderes Intrazellulärmedium verwendet werden kann.

Diese Ausführung mit zwei oder mehr Verbindungskanälen, die über Ventile steuerbar sind, ist grundsätzlich sowohl mit der Ausführung kombinierbar, die zuvor anhand der Fig. 1 bis 4 erläutert wurde, als auch mit der Ausführung gemäß Fig. 6.

**Patentansprüche**

1. Vorrichtung zum elektrischen Messen an Zellen (60; 112) in flüssiger Umgebung (66), mit einem Substrat (34; 86), das von einem Kanal (40; 88; 122) durchsetzt ist, oberhalb dessen eine Zelle (60; 112) mit einer Unterseite (68) ihrer Membran (62) auf einer Oberfläche (49; 85; 128) des Substrates (34; 86) positionierbar ist, ferner mit Mitteln (56) zum Erzeugen eines Druckgefälles (70) entlang des Kanals und mit einer ersten Elektrode (44) sowie mindestens einer zweiten Elektrode (50; 100; 126), die von der ersten Elektrode (44) in Richtung des Kanals (40; 88; 122) beabstandet angeordnet ist, **dadurch gekennzeichnet, dass** die zweite Elektrode (50) an dem von der Unterseite (68) der Membran (62) einer auf der Oberfläche (49; 85; 128) des Substrates (34; 86) positionierten Zelle (60; 112) abgewandten Ende des Kanals (40) angeordnet ist und dass die genannten Elektroden (44, 50; 100; 126) zur elektrischen Abfragung der Zelle (60; 112) geeignet sind, wobei der Kanal (122) an seinem der ersten Elektrode (44) abgewandten Ende über Ventile (118, 120) mit einer Mehrzahl von Kanälen (130, 132) verbunden ist, über die Flüssigkeit ($F_1$, $F_2$) zu- oder abführbar ist.

2. Vorrichtung nach Anspruch 1, **dadurch gekennzeichnet, dass** Mittel (56, 58) zur Steuerung des Druckgefälles (70) vorgesehen sind, sowohl zur Erzeugung eines statischen Druckgefälles (70) zur Einstellung einer Cell-Attached-Konfiguration, als auch zur pulsartigen Erhöhung des Druckgefälles (70) zum Aufreißen der Unterseite (68) der Membran (62).

3. Vorrichtung nach Anspruch 2, **dadurch gekennzeichnet, dass** die zweite Elektrode (50) das abgewandte Ende des Kanals (40) ringförmig umgibt.

4. Vorrichtung nach einem oder mehreren der Ansprüche 1 bis 3, **dadurch gekennzeichnet, dass** eine Vielzahl von Kanälen (40; 88; 122) in einem gemeinsamen Substrat (34; 86; 110) angeordnet ist.

5. Vorrichtung nach einem der Ansprüche 1 bis 4, **dadurch gekennzeichnet, dass** über dem Substrat (34; 86) eine Mikroküvette (12; 84) angeordnet ist, in deren Boden (48; 82) eine Öffnung (49; 88) vorgesehen ist.

**6.** Vorrichtung nach einem der Ansprüche 1 bis 5, **dadurch gekennzeichnet, dass** der Kanal (40; 88; 122) eine lichte Weite (x) von weniger als 10 $\mu$m, vorzugsweise weniger als 5 $\mu$m aufweist.

**7.** Vorrichtung nach einem der Ansprüche 4 bis 6, **dadurch gekennzeichnet, dass** eine Vielzahl von Mikroküvetten (12; 84) in einer Platte (10; 80) angeordnet ist.

**8.** Vorrichtung nach Anspruch 7, **dadurch gekennzeichnet, dass** die Platte (12; 80) mehrschichtig aufgebaut ist.

**9.** Vorrichtung nach Anspruch 8, **dadurch gekennzeichnet, dass** die Platte (10) eine obere Schicht (36), eine mittlere Schicht und eine Unterschicht (32) umfasst, wobei in der oberen Schicht (36) die Mikroküvetten (12) angeordnet sind, die mittlere Schicht das Substrat (34) bildet, in dem die Kanäle (40) angeordnet sind, und die Unterschicht (32) Verbindungskanäle (38) enthält, die zu den Kanälen (40) führen.

**10.** Vorrichtung nach einem der vorhergehenden Ansprüche, **dadurch gekennzeichnet, dass** das Substrat (34; 86) mit einer Unterschicht (32) verbunden ist, die aus einer oder mehreren Schichten aus photostrukturierbaren Materialien besteht, die Verbindungskanäle (38) aufweisen, die zu den Kanälen (40; 88; 122) führen.

**11.** Vorrichtung nach Anspruch 10, **dadurch gekennzeichnet, dass** die Unterschicht (32) auf einen Glasträger aufgebracht ist.

**12.** Vorrichtung nach Anspruch 9, 10 oder 11, **dadurch gekennzeichnet, dass** die Verbindungskanäle (38) eine Breite (b$_2$) zwischen 10 $\mu$m und 40 $\mu$m, vorzugsweise etwa 20 $\mu$m aufweisen.

**13.** Vorrichtung nach einem der Ansprüche 9 bis 12, **dadurch gekennzeichnet, dass** die zweiten Elektroden (50) auf der Unterseite des Substrates (34) oder auf der Oberseite der Unterschicht (32) angeordnet sind.

**14.** Vorrichtung nach Anspruch 13, **dadurch gekennzeichnet, dass** die zweiten Elektroden (50) eine quadratische Fläche mit einer Kantenlänge (a) zwischen 20 $\mu$m und 60 $\mu$m, vorzugsweise etwa 40 $\mu$m aufweisen.

**15.** Vorrichtung nach Anspruch 13 oder 14, **dadurch gekennzeichnet, dass** zu den zweiten Elektroden (50) führende Leiterbahnen (52) zwischen dem Substrat (34) und der Unterschicht (32) angeordnet sind.

**16.** Vorrichtung nach Anspruch 15, **dadurch gekennzeichnet, dass** die Leiterbahnen (52) eine Breite (b$_1$) zwischen 5 $\mu$m und 30 $\mu$m, vorzugsweise etwa 10 $\mu$m aufweisen.

**17.** Vorrichtung nach einem der Ansprüche 9 bis 16, **dadurch gekennzeichnet, dass** zumindest die obere Schicht (36), die Unterschicht (32) oder das Substrat (34) unabhängig voneinander aus Kunststoff, insbesondere aus Polymethylmethacrylat (PMMA), Silikon, PTFE, Polyimid oder aus einem anorganischen Material, insbesondere aus Silizium, Keramik oder Glas bestehen.

**18.** Vorrichtung nach einem der Ansprüche 9 bis 17, **dadurch gekennzeichnet, dass** das Substrat (34; 86) eine Schichtdicke von 2 $\mu$m und 40 $\mu$m, vorzugsweise etwa 5 $\mu$m aufweist.

**19.** Vorrichtung nach einem der Ansprüche 1 bis 18, **dadurch gekennzeichnet, daß** das Substrat (34; 86) aus einer Folie besteht, in der eine Mehrzahl von Kanälen (40; 88) als Bohrungen ausgebildet ist.

**20.** Vorrichtung nach Anspruch 19, **dadurch gekennzeichnet, dass** das Substrat (86) an der Unterseite einer Platte (80) angeordnet ist, in der eine Mehrzahl von Bohrungen als Mikroküvetten (84) ausgebildet ist, deren Boden (82) durch das Substrat (86) gebildet ist, in dem die Kanäle (88) als Löcher ausgebildet sind.

**21.** Vorrichtung nach Anspruch 19 oder 20, **dadurch gekennzeichnet, dass** das Substrat (86) an der Unterseite einer Platte (36) angeordnet ist, in der eine Mehrzahl von Bohrungen als Mikroküvetten (12) ausgebildet ist, in deren Boden (48) Löcher (49) vorgesehen sind, mit denen die Kanäle (40) des Substrates (34) zentriert sind.

**22.** Vorrichtung nach einem der Ansprüche 4 bis 8, **dadurch gekennzeichnet, dass** eine Hydraulik- und Messeinheit (90) vorgesehen ist, die eine zur Unterseite des Substrates (86; 110) hin offene Kammer (92; 124) aufweist, die an der Unterseite des Substrates (86; 110) derart positionierbar ist, dass die Kammer (92; 124) mit einem ausgewählten Kanal (88; 122) kommuniziert und nach außen abgedichtet ist, wobei die Kammer (92; 124) mindestens eine Elektrode (100; 126) enthält und mit mindestens einem Anschlusskanal (96; 130, 132) verbindbar ist, der mit einer Unterdruckquelle verbunden ist.

**23.** Vorrichtung nach Anspruch 22, **dadurch gekennzeichnet, dass** die Kammer (124) mit einer Mehrzahl von Anschlusskanälen (130; 132) über Ventile (118, 120) verbunden ist.

**24.** Vorrichtung nach Anspruch 22 oder 23, **dadurch gekennzeichnet, dass** eine Verfahreinheit (104) zum Verfahren und Positionieren der Platte (80) und der Hydraulik- und Messeinheit (90) relativ zueinander vorgesehen ist.

**Claims**

**1.** Device for carrying out electrical measurements on cells (60; 112) in a liquid environment (66), comprising a substrate (34; 86) which has a channel (40; 88; 122) running through it, above which a cell (60; 112) can be positioned with an underside (68) of its membrane (62) on a surface (49; 85; 128) of the substrate (34; 86), further comprising means (56) to generate a pressure differential (70) along the channel, and comprising a first electrode (44) and also at least one second electrode (50; 100; 126) which is arranged so as to be spaced apart, towards the channel (40; 88; 122), from the underside (68) of the membrane (62), **characterized in that** the second electrode (50) is disposed on that side of the channel (40) which faces away from the underside (68) of the membrane (62) of a cell (60, 112) positioned on the surface (49; 85; 128) of said substrate (34; 86) and **in that** the electrodes (44, 50; 100, 126) are suitable for electrical scanning of the cell (60; 112), whereby the channel (122), at its end facing away from the first electrode (44), is connected via valves (118, 120) to a multiplicity of channels (130, 132) via which the fluid ($F_1$, $F_2$) can be supplied or discharged.

**2.** Device according to Claim 1, **characterized in that** means (56, 58) for controlling the pressure differential (70) are provided, both for the purpose of generating a static pressure differential (70) to establish a cell-attached configuration and for the purpose of a pulse-type increase in the pressure differential (70) to rupture the underside (68) of the membrane (62).

**3.** Device according to Claim 2, **characterized in that** the second electrode (50) annularly surrounds the far end of the channel (40).

**4.** Device according to one or more of Claims 1 to 3, **characterized in that** a multiplicity of channels (40; 88; 122) is disposed in a common substrate (34; 86; 110).

**5.** Device according to any one of Claims 1 to 4, **characterized in that** above the substrate (34; 86) a micro-cuvette (12; 84) is disposed in whose bottom (48; 82) an opening (49; 88) is provided.

**6.** Device according to any one of Claims 1 to 5, **characterized in that** the channel (40; 88; 122) has an inner width (x) of less than 10 $\mu$m, preferably less than 5 $\mu$m.

**7.** Device according to any one of Claims 4 to 6, **characterized in that** a multiplicity of micro-cuvettes 12; 84) are arranged in a plate (10; 80).

**8.** Device according to Claim 7, **characterized in that** the plate (12; 80) has a multilayer structure.

**9.** Device according to Claim 8, **characterized in that** the plate (10) comprises a top layer (36), a middle layer and a bottom layer (32), the micro-cuvettes (12) being disposed in the top layer (36), the middle layer forming the substrate (34) in which the channels (40) are disposed, and the bottom layer (32) containing connecting channels (38) which lead to the channels (40).

**10.** Device according to any one of the preceding claims, **characterized in that** the substrate (34; 86) is bonded to a bottom layer (32) which comprises one or more layers of photopatternable materials which have connecting channels (38) which run to the channels (40; 88; 122).

**11.** Device according to Claim 10, **characterized in that** the bottom layer (32) is applied to a glass mount.

**12.** Device according to Claim 9, 10 or 11, **characterized in that** the connecting channels (38) have a width ($b_2$) of between 10 $\mu$m and 40 $\mu$m, preferably about 20 $\mu$m.

**13.** Device according to any one of Claims 9 to 12, **characterized in that** the second electrodes (50) are disposed on the underside of the substrate (34) or on the top side of the bottom layer (32).

**14.** Device according to Claim 13, **characterized in that** the second electrodes (50) have a square area having an edge length (a) of between 20 $\mu$m and 60 $\mu$m, preferably about 40 $\mu$m.

**15.** Device according to Claim 13 or 14, **characterized in that** conductor tracks (52) which lead to the second electrodes (50) are disposed between the substrate (34) and the bottom layer (32).

**16.** Device according to Claim 15, **characterized in that** the conductor tracks (52) have a width ($b_1$) of between 5 $\mu$m and 30 $\mu$m, preferably about 10 $\mu$m.

**17.** Device according to any one of Claims 9 to 16, **characterized in that** at least the top layer (36), the bottom layer (32) or the substrate (34), independently of one another, are made of plastic, particularly of poly(methyl methacrylate) (PMMA), silicone, PTFE, polyimide or of an inorganic material, particularly of silicon, ceramic material or glass.

18. Device according to any one of Claims 9 to 17, **characterized in that** the substrate (34; 86) has a layer thickness of between 2 μm and 40 μm, preferably about 5 μm.

19. Device according to any one of Claims 1 to 18, **characterized in that** the substrate (34; 86) consists of a sheet in which a multiplicity of channels (40; 88) is fashioned in the form of drilled holes.

20. Device according to Claim 19, **characterized in that** the substrate (86) is disposed on the underside of a plate (80) in which a multiplicity of drilled holes are fashioned as micro-cuvettes (84) whose bottom (82) is formed by the substrate (86) in which the channels (88) are fashioned as holes.

21. Device according to Claim 19 or 20, **characterized in that** the substrate (86) is disposed at the underside of a plate (36) in which a multiplicity of drilled holes are fashioned as micro-cuvettes (12) in whose bottom (48) holes (49) are provided by means of which the channels (40) of the substrate (34) are centred.

22. Device according to any one of Claims 4 to 8, **characterized in that** a hydraulic and measuring unit (90) is provided which has a chamber (92; 124) which is open towards the underside of the substrate (86; 110) and can be positioned at the underside of the substrate (86; 110) in such a way that the chamber (92; 124) communicates with a selected channel (88; 122) and is sealed with respect to the outside, the chamber (92; 124) containing at least one electrode (100; 126) and being connectable to at least one terminal channel (96; 130; 132) which is connected to a negative-pressure source.

23. Device according to Claim 22, **characterized in that** the chamber (124) is connected to a multiplicity of terminal channels (130; 132) via valves (118, 120).

24. Device according to Claim 22 or 23, **characterized in that** a traversing unit (104) for traversing and positioning the plate (80) and the hydraulic and measuring unit (90) relative to one another is provided.

**Revendications**

1. Dispositif pour la mesure électrique de cellules (60 ; 112) en environnement liquide (66), avec un substrat (34 ; 86), qui est traversé par un canal (40 ; 88 ; 122), au-delà duquel une cellule (60 ; 112) avec une face inférieure (68) de leur membrane (62) est positionnable sur une surface (49 ; 85 ; 128) du substrat (34 ;

86), également avec des moyens (56) pour produire une chute de pression (70) le long du canal et avec une première électrode (44) et au moins une deuxième électrode (50 ; 100 ; 126), qui est disposée à distance de la première électrode (44) en direction du canal (40 ; 88 ; 122), **caractérisé en ce que** la deuxième électrode (50) est disposée sur l'extrémité du canal (40) opposée à la face inférieure (68) de la membrane (62) d'une cellule (60 ; 112) positionnée sur la surface (49 ; 85 ; 128) du substrat (34 ; 86) et que les électrodes citées (44, 50 ; 100 ; 126) sont appropriées à l'interrogation électrique de la cellule (60 ; 112), le canal (122) étant lié sur son extrémité opposée à la première électrode (44) par le biais de vannes (118, 120) avec une multitude de canaux (130, 132), par le biais desquels du liquide ($F_1$, $F_2$) peut être amené ou évacué.

2. Dispositif selon la revendication 1, **caractérisé en ce que** des moyens (56, 58) pour commander la chute de pression (70) sont prévus, aussi bien pour produire une chute de pression statique (70) afin d'ajuster une configuration fixée à une cellule, que pour augmenter de manière pulsatoire la chute de pression (70) afin de déchirer la face inférieure (68) de la membrane (62).

3. Dispositif selon la revendication 2, **caractérisé en ce que** le deuxième électrode (50) entoure l'extrémité du canal opposée (40) de manière circulaire.

4. Dispositif selon l'une ou plusieurs des revendications 1 à 3, **caractérisé en ce qu'**une multitude de canaux (40 ; 88 ; 122) est disposée dans un substrat commun (34 ; 86 ; 110).

5. Dispositif selon l'une ou plusieurs des revendications 1 à 4, **caractérisé en ce qu'**une microcuvette (12 ; 84) est disposée sur le substrat (34 ; 86), sur le sol de laquelle (48 ; 82) une ouverture (49 ; 88) est prévue.

6. Dispositif selon l'une ou plusieurs des revendications 1 à 5, **caractérisé en ce** le canal (40 ; 88 ; 122) présente une largeur intérieure (x) de moins de 10 μm, de préférence de moins de 5 μm.

7. Dispositif selon l'une ou plusieurs des revendications 4 à 6, **caractérisé en ce qu'**une multitude de microcuvettes (12 ; 84) est disposée dans une plaque (10 ; 80).

8. Dispositif selon la revendication 7, **caractérisé en ce que** la plaque (12 ; 80) est conçue de manière multicouches.

9. Dispositif selon la revendication 8, **caractérisé en ce que** la plaque (10) comprend une couche supérieure (36), une couche médiane et une sous-couche (32), où les microcuvettes (12) sont disposées dans la couche supérieure (36), la couche médiane forme le substrat (34), dans lequel les canaux (40) sont disposés et la sous-couche (32) comprend des canaux de liaison (38), qui conduisent aux canaux (40).

10. Dispositif selon l'une des revendications précédentes, **caractérisé en ce que** le substrat (34 ; 86) est relié à une sous-couche (32) qui se compose d'une ou de plusieurs couches en matériaux photostructurables, qui présentent des canaux de liaison (38), qui conduisent à des canaux (40 ; 88 ; 122).

11. Dispositif selon la revendication 10, **caractérisé en ce que** la sous-couche (32) est disposée sur un support en verre.

12. Dispositif selon la revendication 9, 10 ou 11, **caractérisé en ce que** les canaux de liaison (38) présentent une largeur ($b_2$) entre 10 $\mu$m et 40 $\mu$m, de préférence d'environ 20 $\mu$m.

13. Dispositif selon l'une des revendications 9 à 12, **caractérisé en ce que** les deuxièmes électrodes (50) sont disposées sur la face inférieure du substrat (34) ou sur la face supérieure de la sous-couche (32).

14. Dispositif selon la revendication 13, **caractérisé en ce que** les deuxièmes électrodes (50) présentent une surface quadratique ayant une longueur d'arête (a) entre 20 $\mu$m et 60 $\mu$m, de préférence d'environ 40 $\mu$m.

15. Dispositif selon la revendication 13 ou 14, **caractérisé en ce que** des voies conductrices (52) conduisant aux deuxièmes électrodes (50) sont disposées entre le substrat (34) et la sous-couche (32).

16. Dispositif selon la revendication 15, **caractérisé en ce que** les voies conductrices (52) présentent une largeur ($b_1$) entre 5 $\mu$m et 30 $\mu$m, de préférence d'environ 10 $\mu$m.

17. Dispositif selon l'une des revendications 9 à 16, **caractérisé en ce qu'**au moins la couche supérieure (36), la sous-couche (32) ou le substrat (34) se composent, indépendamment les uns des autres, de plastique, en particulier de polyméthacrylate de méthyle (PMMA), de silicone, de PTFE, de polyimide ou d'un matériau inorganique, en particulier de silicium, de céramique ou de verre.

18. Dispositif selon l'une des revendications 9 à 17, **caractérisé en ce que** le substrat (34 ; 86) présente une épaisseur de couche de 2 $\mu$m à 40 $\mu$m, de préférence d'environ 5 $\mu$m.

19. Dispositif selon l'une des revendications 1 à 18, **caractérisé en ce que** le substrat (34 ; 86) se compose d'une feuille, dans laquelle une multitude de canaux (40 ; 88) est formée à titre de perforations.

20. Dispositif selon la revendication 19, **caractérisé en ce que** le substrat (86) est disposé sur la face inférieure d'une plaque (80), dans laquelle une multitude de perforations est conçue à titre de microcuvettes (84), dont le sol (82) est formé par le substrat (86), dans lequel les canaux (88) sont conçus sous forme de trous.

21. Dispositif selon la revendication 19 ou 20, **caractérisé en ce que** le substrat (86) est disposé sur la face inférieure d'une plaque (36), dans laquelle une multitude de perforations est conçue à titre de microcuvettes (12), dans le sol desquelles (48) des trous (49) sont formés, avec lesquels les canaux (40) du substrat (34) sont centrés.

22. Dispositif selon l'une des revendications 4 à 8, **caractérisé en ce qu'**une unité hydraulique et de mesure (90) est prévue, qui présente une chambre (92 ; 124) ouverte sur la face inférieure du substrat (86 ; 110), qui peut être positionnée sur la face inférieure du substrat (86 ; 110) de telle sorte que la chambre (92 ; 124) communique avec un canal choisi (88 ; 122) et est étanche vis-à-vis de l'extérieur, la chambre (92 ; 124) comprenant au moins une électrode (100 ; 126) et peut être raccordée avec au moins un canal de connexion (96 ; 130, 132), qui est relié à une source de sous-pression.

23. Dispositif selon la revendication 22, **caractérisé en ce que** la chambre (124) est raccordée à une multitude de canaux de connexion (130 ; 132) par le biais de vannes (118, 120).

24. Dispositif selon la revendication 22 ou 23, **caractérisé en ce qu'**une unité de déplacement (104) pour déplacer et positionner la plaque (80) et l'unité d'hydraulique et de mesure (90) l'une par rapport à l'autre est prévue.

Fig.1

Fig.2

Fig.3

Fig.4

Fig. 5                    STAND DER TECHNIK

EP 1 218 736 B1

Fig.6

Fig.7